# EUROPÄISCHE PATENTSCHRIFT

(11) **EP 0 499 712 B1**
(45) Veröffentlichungstag und Bekanntmachung des Hinweises auf die Patenterteilung: **18.06.1997**
(21) Anmeldenummer: 91121462.5
(22) Anmeldetag: 14.12.1991
(51) Int. Cl.: G01N 21/77, G01N 31/22, C07D 493/10, G01N 21/64, C09B 11/24

(54) **Optischer Sensor**
Optical sensor
Capteur d'optique

(30) Priorität: 22.02.1991 DE 4105531
(43) Veröffentlichungstag der Anmeldung: 26.08.1992
(73) Patentinhaber: Forschungszentrum Karlsruhe GmbH, 76133 Karlsruhe (DE)
(72) Erfinder: Judex, Patricia, W-7505 Ettlingen (DE); Reichert, Johannes, Dr., W-7513 Stutensee (DE); Ache, Hans-Joachim, Prof. Dr., W-7500 Karlsruhe 1 (DE)
(74) Vertreter: Rückert, Friedrich, Dr.

(56) Entgegenhaltungen:
- EP-A- 0 072 627
- EP-A- 0 205 232
- EP-A- 0 214 768
- GB-A- 2 111 491
- US-A- 5 057 431
- ANALYTICAL CHEMISTRY, Bd.35, Nr.8, Juli 1963, COLUMBUS US Seiten 1035 - 1044 D.F.HOEZL WALLACH ; T.L.STECK 'fluorescence techniques in the microdetermination of metals in biological materials'
- ANALYTICAL CHEMISTRY, Bd.56, Nr.4, April 1984, COLUMBUS US Seiten 810 - 813 L.A.SAARI ; W.R.SEITZ 'immobilized calcein for metal ion preconcentration'
- EUROPEAN JOURNAL OF NUCLEAR MEDICINE, Bd.16, Nr.3, 1990 Seiten 137 - 142 K.HORIUCHI ET AL 'LIGANDIN BINDING PHTHALEIN COMPLEXONE COMPLEX OF TECHNETIUM FOR HEPATIC FUNCTION STUDIES'

## Beschreibung

Die Erfindung betrifft einen optischen Sensor gemäß dem Oberbegriff von Anspruch 1, zwei Verfahren zu seiner Herstellung, das Zwischenprodukt Aminocalcein und ein Verfahren zu dessen Herstellung sowie eine Verwendungsmöglichkeit des optischen Sensors.

Aus der Veröffentlichung mit dem Titel "Fluorescence Techniques in the Microdetermination of Metals in Biological Materials" von Donald F. Hoelzl Wallach und Theodore L. Steck, Analytical Chemistry Vol 35, No. 8, July 1963, ist Calcein und seine Verwendbarkeit zur Analyse von Zink(II)-Ionen bekannt. Die Autoren geben für Calcein die folgende Strukturformel an:

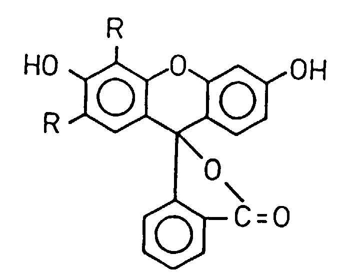

Diese Formel scheint die Struktur von Calcein jedoch nur unzureichend wiederzugeben. Offensichtlich enthält Calcein zwar zwei Iminodiessigsäurereste, doch können deren Stellungen nicht genau lokalisiert werden. Möglicherweise ist Calcein ein isomeres Gemisch von zweifach mit Iminodiessigsäureresten substituiertem Fluorescein.

Im folgenden wird unter Calcein ein im Xanthen-Ringsystem mit zwei Iminodiessigsäureresten substituiertes Fluorescein verstanden, wobei die Stellung der Reste offen bleibt.

Zur Analyse von Zinkionen wurden in der genannten Veröffentlichung Calceinlösungen eingesetzt. Es wurde beobachtet, daß im pH-Bereich zwischen 5.5 und 3,5 die Fluoreszenz von freiem Calcein stark abnimmt, während der Chelatkomplex des Calceins mit Zink(II)-Ionen in diesem pH-Bereich fluoresziert. Die Autoren sind jedoch der Ansicht,daß dieses Phänomen von geringem Nutzen für die Analytik ist, weil die Affinität von Calcein für Zink in diesem pH-Bereich sehr gering ist (vgl. Seite 1039,rechte Spalte). Deshalb wird vorgeschlagen, einen Calcein-Chelatkomplex mit zweiwertigem Kobalt vorzulegen. In einem solchen Komplex kann Zink das Kobalt verdrängen. Somit könnte durch Einstrahlen von Licht und Bestimmen der Intensität des Fluoreszenzlichts auf die Zink-Konzentration geschlossen werden, wenn ein solcher Kobaltkomplex verwendet wird.

Ein solches Verfahren könnte jedoch nicht reversibel durchgeführt werden. Für jede Analyse müßte ein Kobalt-Calceinkomplex vorgelegt und die zu analysierende Lösung zugegeben werden.

Aus der Veröffentlichung mit dem Titel "Immobilized Calcein for Metal Ion Preconcentration" von L. A. Saari und W. R. Seitz, Analytical Chemistry, Vol. 56, No. 4, April 1984 ist ein Sensor der eingangs genannten Art bekannt, bei dem Calcein durch eine chemische Reaktion an eine Matrix aus Cellulose gebunden ist. Die Matrix wird auf das Ende eines sich verzweigenden Lichtleiters aufgebracht. Ein Zweig des Lichtleiters dient der Einstrahlung von Licht einer geeigneten Wellenlänge, während mit dem anderen Zweig die emittierte Fluoreszenzstrahlung transportiert und an dessen Ende gemessen wird. Bei pH-Werten zwischen etwa 5 und 7 ist die Bindung von Übergangsmetallionen an Calcein jedoch so stark, daß die Übergangsmetallionen vollständig aus der Metallionen-Lösung extrahiert werden. Ein solcher Sensor ist unter diesen Bedingungen nicht reversibel. Um immobilisiertes Calcein in einem reversiblen Sensor zu nutzen, schlagen die Autoren vor, bei einem niedrigeren pH-Wert zu arbeiten. Bezüglich der Analyse von Zink(II)-Ionen greifen die Autoren den oben erwähnten Vorschlag auf, mit Co(II)-Ionen beladenes Calcein einzusetzen. Jedoch wäreein solcher Sensor ebenfalls nicht reversibel.

Aus der DE-PS 30 01 669 ist eine Vorrichtung zur Messung von physikalischen Größen und von Stoffkonzentrationen bekannt. Hierfür wird vorgeschlagen, einen durch eine Membran abgetrennten Raum vorzusehen, der einen auf die zu messende Größe optisch ansprechenden Indikatorstoff enthält. Die zu messenden Stofffe durchdringen die Membran und gehen mit dem Indikatorstoff eine Reaktion ein. Das Ausschwemmen des Indikatorstoffes soll verhindert werden, indem der Indikatorstoff unter Verwendung eines Brückenmoleküls covalent an die Membran gebunden wird. Als Membran wird unter anderem Siliconkautschuk vorgeschlagen. Zur Bildung der Brücke zwischen Membran und Indikatorstoff wird ein Silan, vor allem 3-(Triäthoxysilyl)-propylamin verwendet

Aus der EP 0 205 232 A1 ist ein pH-sensitiver Sensor bekannt, bei dem Fluorescein auf poröses Glas aufgebracht ist. Gemäß einem Verfahren zur Herstellung des Sensors wird Aminopropyltriethoxysilan an die Glasoberfläche angekoppelt und die Isothiocyanatgruppe von Fluorescein-Isothiocyanat mit der Aminogruppe des Silans zur Reaktion gebracht.

Aus der EP 0 072 627 A2 ist ein Sensor zur Bestimmung chemischer oder physikalischer Parameter bekannt, bei dem ein Chromophor an poröses Glas angekoppelt ist.

Aufgabe der Erfindung ist, einen Sensor der eingangs genannten Art bereitzustellen, der reversibel ist und dessen Immobilisierungsmatrix chemisch, vor allem in Bezug auf eine Hydrolyse, beständiger ist als die bekannte Matrix.

Die Aufgabe wird erfindungsgemäß durch das kennzeichnende Merkmal des ersten Patentanspruches gelöst. In den abhängigen Ansprüchen sind besonders bevorzugte Ausführungsformen angegeben. In den weiteren Ansprüchen werden Verfahren zur Herstellung des erfindungsgemäßen Sensors sowie das Zwischenprodukt Aminocalcein und ein Verfahren zu dessen Herstellung angegeben.
- Fig. 1: zeigt eine mögliche Meßanordnung
- Fig. 2: zeigt eine Eichkurve für Zink(II)-Ionen.

Die Meßanordnung besteht aus einer Lichtquelle 1, aus der mit Hilfe eines Filters 2 ein monochromatischer Strahl der Wellenlänge 488 nm ausgeblendet wird. Der Strahl trifft innerhalb einer Küvette 5, die die Probelösung enthält, auf den erfindungsgemäßen Sensor 4, der auf einem optisch transparenten Medium 3, beispielsweise einer Glasplatte, mit Hilfe von Silikonkautschuk aufgebracht ist. Die entstehende Fluoreszenzstrahlung der Wellenlänge 508 nm wird nach Durchgang durch einen weiteren Monochromator (nicht dargestellt) mit einem geeigneten Photomultipier 6 und entsprechenden Auswerteeinrichtungen detektiert, wobei die Intensität ein Maß für die Konzentration der Metallionen darstellt.

Selbstverständlich kann auch die von Saari et al vorgeschlagene Meßanordnung verwendet werden.

Wesentlich ist, daß Calcein kovalent, vorzugsweise über ein bewegliches Brückenmolekül, an die Oberfläche von porösem Glas gebunden ist. Diese Maßnahme gewährleistet die Reversibilität des Sensors. Dadurch kann insbesondere auch auf den Einsatz von weiteren Metallionen, mit denen der Sensor vorbeladen wird, verzichtet werden. Das erfindungsgemäße Matrixmaterial ist zudem chemisch beständiger als Cellulose; es wird insbesondere weniger leicht hydrolysiert.

Prinzipiell ist es ausreichend, wenn die Oberflächenstruktur des Glases porös ist. Beispielsweise könnte eine Lichtleiterfaser, deren Ende aus porösem Glas besteht oder auf deren Ende ein poröses Glas aufgebracht ist, verwendet werden, um Calcein chemisch zu binden. Eine andere Möglichkeit besteht darin, poröses Glas, an dessen Oberfläche Calcein immobilisiert ist, auf einem festen, optisch transparenten Träger zu fixieren, wobei dem Träger lediglich die Aufgabe zufällt, dem Sensor eine vorgegebene Form und eine höhere mechanische Beständigkeit zu verleihen.

Als Trägermaterialien kommen insbesondere Glas und optisch transparente Kunststoffe in Betracht. Als Träger können Lichtleiter oder Scheiben verwendet werden, die in eine Probelösung eintauchen. Beispielsweise kann die Glaswand einer geeignet geformten Küvette eingesetzt werden.

Die Verbindung zwischen dem porösen Glas, auf dem Calcein immobilisiert ist, und dem Träger wird vorzugsweise mit Silikonkautschuk hergestellt.

Der erfindungsgemäße Sensor ist zur Analyse von Zink(II)-Ionen besonders geeignet. Die Probelösungen sollen in diesem Fall einen pH-Wert im Bereich zwischen 4 und 7 aufweisen.

Zur Herstellung des erfindungsgemäßen Sensors gibt es zwei Synthesemöglichkeiten, wobei eine der Synthesen von Aminocalcein ausgeht.

Die Erfindung wird im folgenden anhand von Beispielen näher erläutert.

### Beispiel 1:

### Herstellung des Calcein-Sensors (Verfahren I)

Sogenanntes "Controlled Porous Glass" (CPG) mit Poren eines mittleren Durchmessers von 770 nm (77 A) und einer spezifischen Oberfläche von 182 m²/g wird mit Glycidoxypropyl-Triethoxysilan silanisiert (Schritt a). Hierzu wurden 3,5 g CPG unter Rühren in 150 ml Toluol in Stickstoffatmosphäre auf 80° C erhitzt. Nach Zugabe von 1,5 g des Silans wurde bei 80° vier Stunden lang gerührt. Schließlich wurd das CPG abfiltriert, mit 50 ml Toluol und 100 ml Aceton gewaschen und bei 90° C getrocknet. Das physisobierte Silan wurde durch 4-tägige Heißextraktion mit Methanol entfernt.

Anschließend wurde das Epoxid durch eintägiges Rühren des silanisierten CPG in 2 n HCl sauer hydrolysiert (Schritt b), wobei sich ein Diol bildet.Das Produkt wurde abfiltriert, mit 100 ml bidestilliertem Wasser sowie 100 ml Methanol gewaschen und bei 70 C getrocknet. Kommerziell erhältliches Calcein wurde durch eine Friedels-Craft-Alkylierung mit dem Diol auf dem CPG umgesetzt (Schritt c). Hierzu wurden 467 mg Calcein in 50 ml Eisessig bei 100 C gelöst. Es wurden 353 mg des Diols aus Schritt b und 10 ml konzentrierte Schwefelsäure zugesetzt und 12 Stunden bei 100 C gerührt. Die Reaktionsmischung wurde auf 200 ml Eiswasser gegossen. Aschließend wurde das Produkt abfiltriert, mit 200 ml bidestilliertem Wasser gewaschen und 3 Tage mit Aceton heißextrahiert.

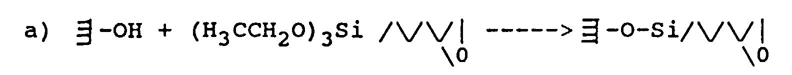

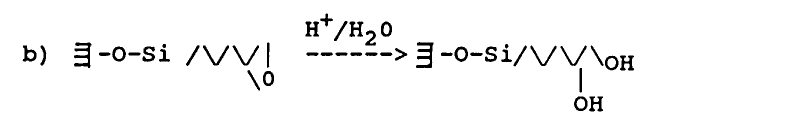

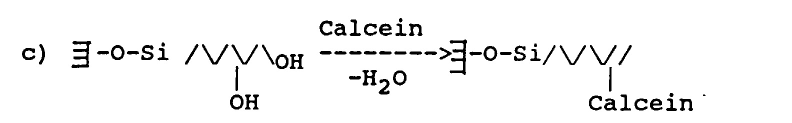

### Beispiel 2:

### Herstellung des Calcein-Sensors (Verfahren II)

Sogenanntes "Controlled Porous Glas" (CPG) mit Poren eines mittleren Durchmessers von 770 nm (77 A) und einer spezifischen Oberfläche von 182 m2/g wird mit N-(Aminoethyl)-Aminopropyl-Triethoxysilan silanisiert (Schritt a). Hierzu werden 3,5 g CPG in 150 ml Toluol p. a. unter Rühren auf 80° C erhitzt. Die Suspension wurde mit trockenem Stickstoff gespült, bis die Reaktionstemperatur erreicht war. Nach Zugabe von 1,632g des Silans wurde bei 80° C vier Stunden lang gerührt. Das CPG wurde abfiltriert, mit 50 ml Toluol und 100 ml Aceton gewaschen und bei 90° C vier Stunden lang getrocknet. Das physisorbierte Silan wurde durch 4-tägige Heißextraktion mit Methanol entfernt.

Die auf dem CPG chemisch fixierten Aminogruppen werden mit Thiophosgen in Phosphatpuffer pH 6,88 zu Isothiocyanatgruppen umgesetzt (Schritt b). Das nach den Schritten a und b grenzflächenmodifizierte CPG wird mit Aminocalcein zur Reaktion gebracht, wobei ein Thioharnstoffderivat entsteht (Schritt c).

Hierzu wurden 37,6 mg Aminocalcein in 5 ml Phosphatpuffer pH 8,0 gelöst. Es wurden 68,8 mg des nach Schritt b erhaltenen Produkts zugegeben und 30 Stunden bei Raumtemperatur gerührt. Das Produkt wurde abfiltriert, mit 500 ml bidestilliertem Wasser gewaschen und 3 Tage mit Aceton heißextrahiert.

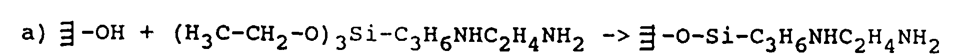

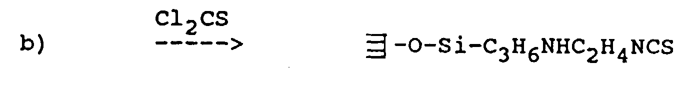

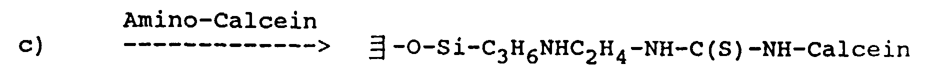

### Beispiel 3:

### Herstellung von Aminocalcein

7,3 g (0,02 mol) Aminofluorescein werden in 20 ml 60%igem Ethanol gelöst. Es werden 6 ml 30%ige Natronlauge zugegeben (Lösung 1).
10,6 g (0,08 mol) Iminodiessigsäure werden in 15 ml destilliertem Wasser gelöst und mit 12 ml 30%iger Natronlauge gelöst (Lösung 2).

Die Lösungen 1 und 2 werden auf 4° C gekühlt und unter Rühren vermischt.

Anschließend werden 7,5 g 37%iges Formaldehyd tropfenweise unter Rühren zugegeben, wobei die Temperatur der Reaktionsmischung unter 4° C gehalten wird.

Die Reaktionsmischung wird danach auf 70° C erwärmt und 8 Stunden bei 70° C gerührt. Nach dem Abkühlen auf Raumtemperatur wird auf 1000 ml verdünnt und mit 3,5 n Salzsäure bis zum Ausfallen des Rohprodukts versetzt (pH ca. 2,5). Der Niederschlag wird abfiltriert und mit 2 l Wasser gewaschen und an der Luft trocknen gelassen.

Zur Reinigung wird das Rohprodukt in 50 ml heißem 70%igem Ethanol gelöst und sofort filtriert. Das Produkt fällt nach dem Abkühlen des Filtrats aus. Es wird abgetrennt und 3 mal aus 70%igem Ethanol umkristallisiert.

### Beispiel 4:

### Aufbringen des Produkts aus den Beispielen 1 oder 2 auf einen Träger

Das Ende eines Lichtleiters oder eine Glasplatte als optisch transparente Träger wurden mit Siliconkautschuk bestrichen. Nach einer Wartezeit von 35 Minuten wird das Glaspulver mit dem immobilisierten Calcein aufgestreut. Man läßt ca 12 Stunden aushärten und entfernt das überschüssige, nicht haftende CPG.

Die folgenden Beispiele wurden mit den nach Beispiel 1 und 2 erhaltenen Sensoren, die gemäß Beispiel 4 auf eine Glasplatte aufgetragen wurden, durchgeführt.

### Beispiel 5:

### Nachweis der Reversibilität des erfindungsgemäßen Sensors

In einer Meßanordnung gemäß Fig. 1 wurde der erfindungsgemäße Sensor wechselweise mit einer Zn(II)-Ionen enthaltenden Lösung und mit einer Blindlösung in Kontakt gebracht. Die Blindlösung bestand aus einer Pufferlösung mit einem pH von 4,7. Die Versuchsergebnisse sind in der folgenden Tabelle zusammengefaßt:

| Konzentration von Zn(II) in ppm | Intensität des Fluoreszenzlichts (508 nm) | Meßzeit in min. |
|---|---|---|
| 0 | 84,67 | 30 |
| 1 | 113,34 | 5 |
| 0 | 85,69 | 25 |
| 1 | 110,27 | 5 |
| 0 | 83,64 | 25 |
| 1 | 109,29 | 5 |
| 0 | 88,76 | 25 |
| 1 | 114,12 | 5 |

Meßbedingungen:

| | | |
|---|---|---|
| Spaltbreite | Anregung (488 nm): | 1,5 nm |
| | Emission (508 nm): | 3,0 nm |
| pH-Wert der Zn-Lösung: | | 4,7 |
| Nachweisgrenze (3 Sigma): | | 0,5 ppm |
| Meßfehler: | | 3 % |

### Beispiel 6:

In-line-Messungen mit dem erfindungsgemäßen Sensor und Zn(II)-Ionen enthaltenden Lösungen

| | | | | | | | |
|---|---|---|---|---|---|---|---|
| Konz. von Zn(II) in mg/l: | 1 | 5 | 10 | 1 | 5 | 1 | etc. |
| Meßzeit in Minuten: | 2 | 2 | 2 | 5-7 | 2 | <5 | |

Der erfindungsgemäße Sensor erwies sich über einen Zeitraum von 3 Tagen als stabil. Die Eichkurve gemäß Fig.2 gibt die Mittelwerte über diesen Zeitraum an. Danach muß der Sensor nachkalibriert werden. Im folgenden werden die Meßergebnisse zusammengestellt:

| | |
|---|---|
| Meßfehler der Eichkurve:ca. | 3 % |
| Nachweisgrenze: | 0,5 mg Zn(II)/l |
| Empfindlichkeit: | 25 Einheiten/mg Zn(II)/l |
| Ansprechzeit: | 2 min. |
| Abklingzeit: | abhängig von der Zn(II)-Konzentration in der Lösung; max. 30 min., wenn der Sensor im Sättigungsbereich war und bis zum Blindwert gemessen wurde. |

Die Vorteile des erfindungsgemäßen Sensors können wie folgt zusammengefaßt werden:

Der Sensor läßt eine sehr kompakte Bauweise zu; er ist insbesondere für in-line Messungen an schwer zugänglichen Orten geeignet. Die Ansprechbarkeit ist über Tage stabil; dieser Zeitraum läßt sich durch Nachkalibrieren noch verlängern. Ein weiterer Vorteil ist die leichte Austauschbarkeit und der geringe Aufwand für periphere Geräte (Lichtquelle, Monochromator, Photomultipier mit Auswerteeinheit), wodurch sich ein kostengünstiges Meßverfahren ergibt. Zudem zeigt die Sensor-Matrix etwa die gleiche chemische Beständigkeit wie ein Lichtleiter.

## Patentansprüche

1. Optischer Sensor zur Messung der Konzentration von Metallionen,
a) der Calcein enthält,
b) das durch eine chemische Reaktion an eine Matrix gebunden und
dadurch immobilisiert ist,
dadurch gekennzeichnet, daß
c) die Matrix ein Glas mit poröser Oberflächenstruktur ist.

2. Optischer Sensor nach Anspruch 1, dadurch gekennzeichnet, daß die Matrix ein poröses Glas mit einem mittleren Porendurchmesser von 7,7 bis 100 nm und einer spezifischen Oberfläche von 25 bis 200 m²/g darstellt.

3. Optischer Sensor nach Anspruch 2, dadurch gekennzeichnet, daß das Glas auf einem festen, optisch transparenten Medium aufgebracht ist.

4. Verfahren zur Herstellung des optischen Sensors gemäß Anspruch 1 oder 2, dadurch gekennzeichnet, daß
a) das Glas mit einem Aminoalkyl-Alkoxysilan silanisiert wird,
b) die dabei auf dem Glas chemisch fixierten Aminogruppen mit Thiophosgen zu Isothiocyanatgruppen umgesetzt werden,
c) die Isothiocyanatgruppen mit Aminocalcein zu Thioharnstoffderivaten umgesetzt werden.

5. Verfahren zur Herstellung des optischen Sensors gemäß Anspruch 1 oder 2, dadurch gekennzeichnet, daß
a) das Glas mit einem Epoxyalkyl-Alkoxysilan silanisiert wird,
b) die Epoxygruppe sauer hydrolysiert wird, wobei ein Diol entsteht,
c) Calcein durch eine Friedel-Crafts-Alkylierung mit dem Diol umgesetzt wird.

6. Verfahren zur Herstellung des optischen Sensors gemaß Anspruch 3, dadurch gekennzeichnet, daß das Glas mit Hilfe von Silikonkautschuk auf dem festen, optisch transparenten Medium aufgebracht wird.

7. Aminocalcein der Formel 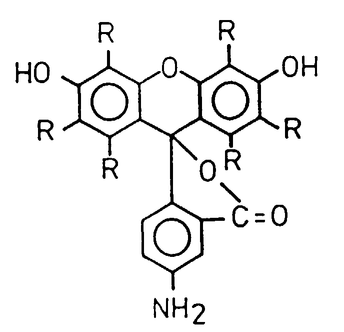 wo zwei der Reste R jeweils einen Iminodiessigsäurerest [-CH₂-N(CH₂COOH)₂] und die übrigen Wasserstoff bedeuten.

8. Verfahren zur Herstellung von Aminocalcein gemäß Anspruch 7, dadurch gekennzeichnet, daß in an sich bekannter Weise Aminofluorescein mit Iminodiessigsäure umgesetzt wird.

9. Verwendung des optischen Sensors gemäß Patentanspruch 1,2 oder 3 zur Bestimmung der Konzentration von Zink(II)-Ionen in einem Verfahren, bei dem der optische Sensor mit Licht bestrahlt wird und aus der Intensität des Fluoreszenzlichts die Konzentration ermittelt wird.

## Claims

1. Optical sensor, for the measurement of the concentration of metal ions,
a) which sensor contains calcein,
b) which calcein is bonded to a matrix by a chemical reaction and is thereby immobilised,
characterised in that
c) the matrix is a glass having a porous surface structure.

2. Optical sensor according to claim 1, characterised in that the matrix represents a porous glass having a mean pore diameter of between 7.7 and 100 nm and a specific surface of between 25 and 200 m²/g.

3. Optical sensor according to claim 2, characterised in that the glass is applied to a solid, optically transparent medium.

4. Method for the production of the optical sensor according to claim 1 or 2, characterised in that
a) the glass is silanised with an aminoalkylalkoxysilane,
b) the amino groups, chemically fixed thereby on the glass, are converted to isothiocyanate groups by thiophosgene, and
c) the isothiocyanate groups are converted to thiourea derivatives by aminocalcein.

5. Method for the production of the optical sensor according to claim 1 or 2, characterised in that
a) the glass is silanised with an epoxyalkylalkoxysilane,
b) the epoxy group is acidly hydrolysed, whereby a diol is produced, and
c) calcein is converted by a Friedel Crafts' alkylisation with the diol.

6. Method for the production of the optical sensor according to claim 3, characterised in that the glass is applied to the solid, optically transparent medium by means of silicone rubber.

7. Aminocalcein of the formula 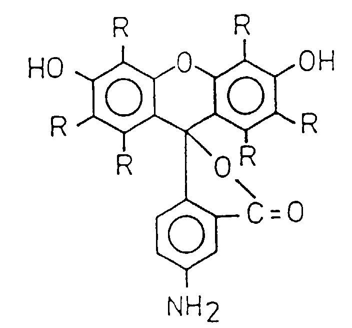 where two of the radicals R each signify an iminodiacetic acid radical [-CH₂-N(CH₂COOH)₂], and the other radicals signify hydrogen.

8. Method for the production of aminocalcein according to claim 7, characterised in that aminofluorescein is converted by iminodiacetic acid in a manner known per se.

9. Use of the optical sensor according to claim 1, 2 or 3 for the determination of the concentration of zinc(II) ions in a method wherein the optical sensor is irradiated with light, and the concentration is determined from the intensity of the fluorescent light.

## Revendications

1. Capteur optique pour mesurer la concentration d'ions métalliques,
a) qui contient de la calcéïne,
b) qui est liée par une réaction chimique à une matrice et qui est ainsi immobilisée,
caractérisé en ce que
c) la matrice est un verre à structure superficielle poreuse.

2. Capteur optique selon la revendication 1,
caractérisé en ce que
la matrice est en verre poreux ayant un diamètre moyen de pore de 7,7 à 100 nm et a une surface spécifique de 25 à 200 m²/g,

3. Capteur optique selon la revendication 2,
caractérisé en ce qu'on dépose le verre sur un milieu solide optiquement transparent.

4. Procédé de préparation du capteur optique selon la revendication 1 ou 2,
caractérisé en ce que
a) on silanise le verre avec un aminoalkyl-alcoxysilane,
b) on fait réagir les groupes amino fixés chimiquement sur le verre avec du thiophosgène pour donner des groupes isothiocyanates
c) on fait réagir les groupes isothiocyanates avec l'aminocalcéïne pour donner des dérivés thiourées.

5. Procédé de préparation du capteur optique selon la revendication 1 ou 2,
caractérisé en ce que
a) on silanise le verre avec un époxy-alkyl-alcoxysilane,
b) on hydrolyse le groupe époxy en milieu acide, ce qui donne un diol,
c) on fait réagir la calcéïne par une réaction d'alkylation de Friedel-Crafts avec un diol.

6. Procédé de préparation du capteur optique selon la revendication 3,
caractérisé en ce qu'
on fixe le verre à l'aide de caoutchouc au silicone sur le milieu solide optiquement transparent.

7. Aminocalcéïne de formule 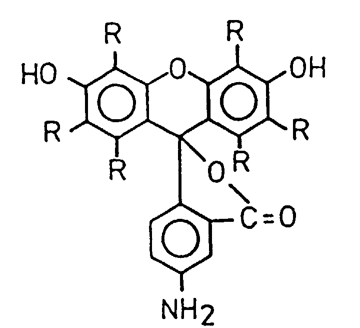 ou deux des radicaux R représentent respectivement un radical iminodiacétiques [-CH₂-N(CH₂COOH)₂] et les autres représentent de l'hydrogène.

8. Procédé de préparation d'aminocalcéïne selon la revendication 7,
caractérisé en ce qu'
on fait réagir de manière déjà connue l'aminofluorescéïne avec l'acide iminodiacétique.

9. Utilisation du capteur optique selon la revendication 1, 2 ou 3 pour mesurer la concentration d'ions zinc (II) dans un procédé où on isole le capteur optique avec de la lumière et où on détermine la concentration à partir de l'intensité de la lumière fluorescente.
